(19) Europäisches Patentamt
European Patent Office
Office européen des brevets

(11) Publication number: **0 408 749 A1**

(12) **EUROPEAN PATENT APPLICATION published in accordance with Art. 158(3) EPC**

(21) Application number: **89903944.0**

(22) Date of filing: **30.11.88**

(86) International application number: **PCT/SU88/00252**

(87) International publication number: **WO 90/06310 (14.06.90 90/14)**

(51) Int. Cl.5: **C07D 493/10**, C07D 307/94, A61K 9/08, A61K 35/78, A61K 31/19, A61K 31/34

(43) Date of publication of application: **23.01.91 Bulletin 91/04**

(84) Designated Contracting States: **BE CH DE FR GB IT LI**

(71) Applicant: **TASHKENTSKY GOSUDARSTVENNY UNIVERSITET IMENI V.I. LENINA**
**Vuzgorodok**
**Tashkent, 700095(SU)**

Applicant: **INSTITUT BIOORGANICHESKOI KHIMII AKADEMII NAUK UZBEXKOI SSR**
**pr. M. Gorkogo, 83**
**Tashkent, 700143(US)**

(72) Inventor: **ZAINUTDINOV, Umardzhan Nasrutdinovich**
**ul. Kashgary, tupik 4-6**
**Tashkent, 700133(SU)**
Inventor: **NURMATOVA, Mubarak P. ul. Khadzhaeva, proezd 1-6**
**Kalininsky raion Tashkentskaya obl. Sovkhoz"Khaskozvo", 702043(SU)**
Inventor: **ASLANOV, Khakim**
**Massiv Kara-kamysh, 2/4-27-15**
**Tashkent, 700178(SU)**
Inventor: **SADYKOV, Abid**
**deceased**
**(SU)**
Inventor: **ABDURAKHMANOV, Tair**
**2 Tupik B.Khmelnitskogo, 8a**
**Andizhan, 710006(SU)**
Inventor: **NASIROV, Sanzhar Khaidarovich**
**Massiv Karasu-2, 25-4**
**Tashkent, 700197(SU)**
Inventor: **ISLAMOV, Rakhim**
**2 Proezd Zhukovskogo, 19-4**
**Tashkent, 700047(SU)**
Inventor: **DANILCHUK, Dmitry Nikolaevich**
**ul. K.Marxa, 8-12**
**Chimkent, 486000(SU)**
Inventor: **YANKOVSKY, Boris Andreevich**
**ul. Mangeldina, 36-106**
**Chimkent, 486005(SU)**
Inventor: **ZAKHAROV, Vadim Pavlovich**
**pr. 60 let Oktyabrya, 25-1-15**
**Moscow, 117036(SU)**

(74) Representative: **Knott, Stephen Gilbert et al**
**MATHISEN, MACARA & CO. The Coach House 6-8 Swakeleys Roadoad**
**Ickenham Uxbridge Middlesex UB10 8BZ(GB)**

(54) **DERIVATIVES OF 3,18-DIHYDROXY-9,13-EPOXYLABDANE, METHOD OF OBTAINING THEM AND A HEMOSTATIC PHARMACEUTICAL PREPARATION BASED THEREON.**

(57)

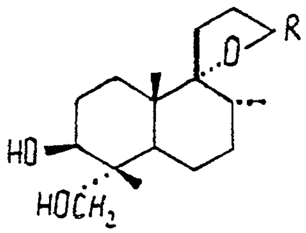

(I)

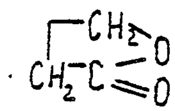

(a)

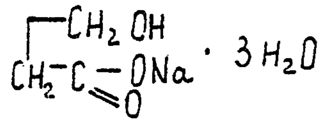

(b)

New compositions which are derivatives of 3,18-dihydroxy-9,13-epoxylabdane of general formula (I), where R = (a); (b). A method of obtaining said derivatives of 3,18-dihydroxy-9,13-epoxylabdane consists in subjecting the 3,18-dihydroxy-9,13-epoxylabdane to the dehydration in the medium of an organic solvent at the latter's boiling temperatures in the presence of a catalyst and in subsequently extracting the desired product. The proposed compositions have hemostatic properties. A hemostatic pharmaceutical preparation comprises, as an active component, the trihydrate of the sodium salt of 3,16,18-trihydroxy-9,13-epoxlylabdane-15-acid.

TITLE MODIFIED see front page

# DERIVATIVES OF 3,18-DYHYDROXY-9,13-EPOXYLABDANE, METHOD FOR PREPARING SAME AND A HEMOSTATIC PHARMACEUTICAL PREPARATION BASED THEREON

## Field of the Invention

The present invention relates to the art of organic chemistry and; more particularly, to novel derivatives of 3,18-dyhydroxy-9,13-epoxylabdane, a method for preparing same and a pharmaceutical preparation exhibiting a hemostatic effect, based thereon, and useful in medicine.

## Prior Art

Known in the art are various compounds exhibiting a hemostatic effect such as sodium 2,3-dihydro-2-methyl-1,4-naphthoquinone-2-sulphonate, dicynone (2,5-dihydroxybenzenesulphonate-3-diethylammonium salt) /M.D.Mashkovsky "Pharmaceutical Preparations", 1984, part 1, part 2, "Meditsina" Publishing House, p. 39, p. 472/.

However, sodium 2,3-dihydro-2-methyl-1,4-naphthoquinone-2-sulphonate is effective only in the case of synthesys of the liver prothrombin and it cannot be used in acute hemorrhages, since its hemostatic effect is noted within 12-18 hours after its administration to patients.

Described in the literature is a compound which is a derivate of 3,18-dihydroxy-9,13-epoxylabdane, namely 3,15,16,18-tetrahydroxy-9,13-epoxylabdane:

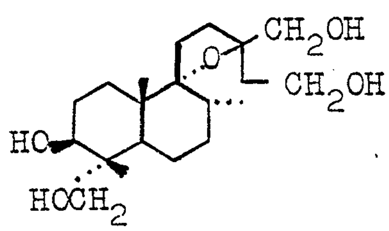

("Izvestia AN SSSR, seria "Khimia", No. 9, 1970, "Nauka" Publishers, Moscow, O.S.Chizhov et al., "Structure of Lagochiline", p. 1983).

However, this compound is insoluble in water and in oils, but soluble in ethanol. This is responsible for its weak hemostatic effect, hinders its wide application in medicine, because it does not enable its administration in such a rapid effect form as local treatment of bleeding spots or injections, especially necessary in the case of inner hemorrhages.

Known in the art is a method for preparing derivatives of 3,18-dihydroxy-9,13-epoxylabdane, namely: 3,15,16,18-tetrahydroxy-9,13-epoxylabdane (SU, A, 99459).

According to this method; 3,15,16,18-tetrahydroxy-9,13-epoxylabdane is obtained by extracting an above ground part of the plant Lagochilus inebrians by means of dichloroethane at a temperature of 80-85°C, followed by isolation of the desired product.

This method, however, does not make it possible to produce other derivatives of 3,18-dihydroxy-9,13-eposylabdane.

## Disclosure of the Invention

The compounds according to the present invention, the method for preparing same and the pharmateutical preparation based thereon are novel and hitherto unknown in the literature.

The present invention is based on the problem of providing novel compounds possessing an increased hemostatic activity, developing of a method for preparing same which makes it possible to prepare compounds useful for medical applications.

This object is accomplished by that according to the present invention the novel compounds, viz. derivatives of 3,18-dihydroxy-9,13-epoxylabdane have the following general formula:

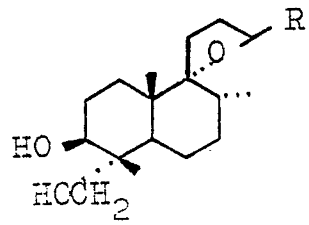

wherein:

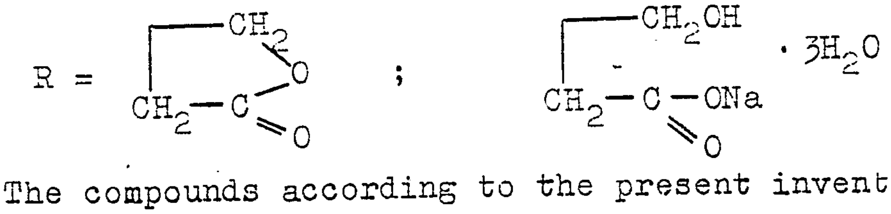

The compounds according to the present invention exhibit an increased hemostatic activity.

The method for preparing the compounds according to the invention - derivatives of 3,18-dihydroxy-9,13-epoxylabdane comprises dehydrogenation of 3,15,16,18-tetrahydroxy-9,13-epoxylabdane in the presence of a catalyst in a medium of a boiling organic solvent, followed by isolation of the desired product.

To increase the yield of the desired product, it is advisable that 3,15,16,18-tetrahydroxy-9,13-epoxylabdane be treated with acetone in the presence of a 2% aqueous solution of sulfuric acid prior to said dehydrogenation.

Furthermore, for acceleration of the dehydrogenation process, it is desirable that activated Raney nickel be used as the dehydrogenation catalyst.

As the organic solvent it is advisable to use toluene.

The present invention also relates to a hemostatic-effect pharmaceutical preparation comprising an active principle and a pharmaceutically acceptable solvent which, according to the present invention, contains as the active principle 3,16,18-trihydroxy-9,13-epoxylabdan-15-ic acid sodium salt trihydrate of the following formula:

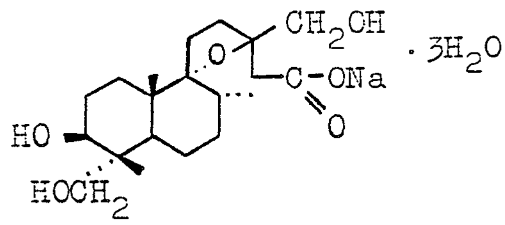

The pharmaceutical preparation can be used in various preparative forms. Preferably it is administered in the form of injection solutions with a content of the active principle of 0.4 to 0.6% by mass. The pharmaceutical preparation should preferably contain bidistilled water as the pharmaceutically acceptable solvent.

Best Mode for Carrying out the Invention

The compounds according to the present invention, i.e. derivatives of 3,18-dihydroxy-9,13-epoxylabdane are crystalline white powders.

Apart from this, 3,18-dihydroxy-9,13-epoxylabdan-15,16-olide is well soluble in organic solvents and oils, while 3,16,18-trihydroxy-9,13-epoxylabdan-15-ic acid sodium salt trihydrate is well soluble in water, it is also soluble in ethanol upon heating.

For all the compounds IR- and PMR-spectra were recorded.

The IR-spectra of the compounds according to the present invention were taken in KBr.

The IR-spectrum of 3,18-dihydroxy-9,13-epoxylabdan-15,16-olide has absorption bands within the range of 3,2000-3,400 $cm^{-1}$ (characteristic for hydroxy groups), at 1,790 $cm^{-1}$ (characteristic for a five-membered lactone cycle), at 2,890-2,930 $cm^{-1}$ (characteristic for methylene and methine groups).

The IR-spectrum of 3,16,18-trihydroxy-9,13-epoxylabdan-15-ic acid sodium salt trihydrate has absorption bands within the range of 3,300-3,500 $cm^{-1}$ (characteristic for hydroxy groups), 1,550-1,610 $cm^{-1}$ and 1,400 $cm^{-1}$ (characteristic for carbonyl group), within the range of from 2,850 to 2,950 $cm^{-1}$ -characteristic for methylene and methine groups). In the PMR-spectrum of 3,18-dihydroxy-9,13-epoxylabdan-15,16-olide there were observed:

$CH_3$ at $C_4$ = 1.0 (ppm) (singlet)
$CH_3$ at $C_{10}$ = 0.93 (ppm) (singlet)
$CH_3$ at $C_{17}$ = 0.74 (ppm) (duplet)
12-$CH_2$ = 1.2-2.2 (ppm) (multiplet)

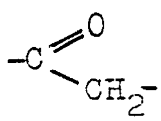 = 2.35-3.0 (ppm) (quartet)

$-CH_2-O-$ = 4.0-4.4 (ppm) (quartet)

The method according to the present invention is performed in the following manner.

Charged into a reactor are 3,15,16,18-tetrahydroxy-9,13-epoxylabdane, an organic solvent (benzene, toluene, ketone and the like) preferably toluene abd a dehydration catalyst preferably Raney nickel. The process is conducted at the boiling temperature of said solvent.

On completion of the reaction of the catalytical dehydrogenation the desired product is recovered in the following manner. The catalyst is filtered-off, the filtrate is treated with caustic soda and then with a concentrated sulphuric acid to an acidic reaction. The formed precipitate is extracted with an organic solvent, such as diethyl ether. The extract is washed to the neutral reaction and the residue after distilling-off said diethyl ether is recrystallized to give the desired product - 3,18-dihydroxy-9,13-epoxylabdan-15,16-olide.

The recovery of the desired product in the form of 3,16,18-trihydroxy-9,13-epoxylabdan-15-ic acid sodium salt trihydrate is carried out on the basis of 3,18-dihydroxy-9,13-epoxylabdan-15-16-olide: a solution of 3,18-dihydroxy-9,13-epoxylabdan-15,16-olide in ethanol is alkalinified with an aqueous solution of caustic soda to the pH = 9 , the solvent is distilled-off, the residue is recrystallized from ethanol.

It is advisable, prior to carrying out the process of the catalytical dehydrogenation, the treat 3,15,16,18-tetrahydroxy-9,13-epoxylabdane with acetone in the presence of a 2% aqueous solution of sulphuric acid.

The resulting compounds - derivatives of 3,18-dihydroxy-9,13-epoxylabdane of the following general formula:

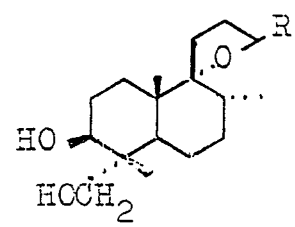

wherein:

R = 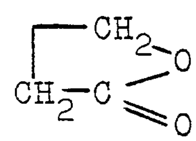 ; 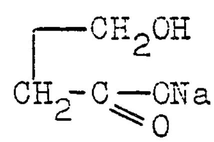 · $3H_2O$

exhibit a hemostatic activity.

The pharmacological studies of the hemostatic activity of the compounds according to the present invention was conducted in vivo and in vitro with the use of biochemical tests and by means of a thromboelastograph.

The experiments were carried out with dogs, rabbits, white rats by may of a single-time administration of the compound orally, intravenously and hypodermally in the doses of 1, 5, 10, 25 and 50 mg/kg of the animal's bodymass. The blood for examination was taken from an opened dog's thigh vein prior to administration of the compound and 10, 30, 60, 90 and 120 min thereafter. For the cuvette of the thromboelastograph the blood was taken in the amount of 0.36 ml, while for biochemical tests -up to 10 ml (as the conservant use was made of a 1.34% solution of sodium oxalate which was added in the ratio of 1:9).

The compounds according to the present invention introduced intravenously in the dose of 1 and 5 mg/kg of the dog's bodymass did not substantially change the thromboelastogram parameters.

In another series of experiments the compound according to the present invention - 3,16,18-trihydroxy-9,13-epoxylabdan-15-ic acid sodium salt trihydrate was administered in the dose of 10 mg/kg of the animal's bodymass; it was shown that this compound promoted the process of blood coagulation in dogs.

The next series of experiments - administration of the compound according to the present invention in the dose of 25 mg/kg of the animals' bodymass. The obtained data have been statistically processes and are shown in Table 1 hereinbelow.

Table 1

Effect of 3,16,18-trihydroxy-9-13-epoxylabdan-15-ic acid sodium salt trihydrate on the parameters of a dog thromboelastogram (dose 25 mg/kg of the animal's bodymass, a group of 10 animals)

| Parameters | Time of reaction of blood coagulation, R, mm | | | | |
|---|---|---|---|---|---|
| | 1 | 2 | 3 | 4 | 5 |
| Arithmetic mean, M | 23.0 | 10.0 | 12.0 | 9.0 | 11.0 |
| Mean square deviation | 8.70 | 3.00 | 3.50 | 2.20 | 2.68 |
| Mean error of arithmetic mean | 2.10 | 1.10 | 1.20 | 0.80 | 0.92 |

Table 1 (continued)

| 1 | I | 2 | 3 | 4 | 5 |
|---|---|---|---|---|---|
| Specific coagulation constant $t = \frac{M_1 - M_2}{\sqrt{m_1^2 + m_2^2}}$, mm | - | 5.40 | 4.50 | 6.35 | 5.40 |
| Certainty of the experiments, $\rho$ | - | <0.0I | <0.0I | <0.0I | <0.0I |

Table 1 (continued)

| Parameters | Time of the clot formation K,mm | | | | |
|---|---|---|---|---|---|
| Arithmetic mean, M | 20.0 | I2.0 | II.0 | I0.0 | 9.0 |
| Mean square deviation, $\pm\sigma$ | 8.4 | 2.6 | 3.2 | 2.2 | 2.2 |
| Mean error of the arithmetic mean | 2.8 | 0.9 | 0.8 | 0.8 | I.I |
| Specific constant of coagulation $t = \frac{M_1 - M_2}{\sqrt{m_1^2 + m_2^2}}$,mm | 2.0 | 2.6 | 3.I | 3.4 | 4.0 |
| Certainty of the experiments, $\rho$ | <0.0I | <0.0I | <0.0I | <0.0I | <0.0I |

Table 1 (continued)

| Parameters | Total constant of blood coagulation, T, mm | | | | |
|---|---|---|---|---|---|
| | I | 2 | 3 | 4 | 5 |
| Arithmetic mean, M | II9.0 | 90.0 | 97.0 | 80.0 | 94.0 |
| Mean square deviation, $\pm\sigma$ | 25.0 | 2I.0 | 26.0 | 20.0 | 40.0 |
| Mean error of the arithmetic mean, $\pm m$ | 9.6 | 8.7 | 9.3 | 6.9 | I5.0 |

Table 1 (continued)

| | I | 2 | 3 | 4 | 5 |
|---|---|---|---|---|---|
| Specific coagulation constant $t = \frac{M_1 - M_2}{\sqrt{m_1^2 + m_2^2}}$, mm | I.0 | I.5 | I.6 | 3.2 | I.3 |
| Certainty of the experiments, $\rho$ | - | < 0.5 | < 0.5 | < 0.5 | < 0.5 |

Table 1 (continued)

| Parameters | Maximum amplitude reflecting the clot elasticity, mm | | | | |
|---|---|---|---|---|---|
| | I | 2 | 3 | 4 | 5 |
| Arithmetic mean, M | 70.0 | 70.0 | 73.0 | 70.0 | 70.0 |
| Mean square deviation, $\pm \sigma$ | 8.I | II.6 | 9.7 | I2.5 | 7.3 |
| Mean error of the arithmetic mean, $\pm m$ | 3.3 | 4.2 | 3.3 | 4.2 | 2.6 |
| Specific constant of coagulation, $t = \frac{M_1 - M_2}{\sqrt{m_1^2 + m_2^2}}$, mm | - | 0 | 0 | 0 | 0 |
| Certainty of the experiments, $\rho$ | - | 0 | 0 | 0 | 0 |

Table 1 (continued)

| Parameters | Duration of blood coagulation, R + K, mm | | | | |
|---|---|---|---|---|---|
| | I | 2 | 3 | 4 | 5 |
| Arithmetic mean, M | 43.3 | 20.I | 22.7 | I8.8 | I8.I |
| Mean square deviation, $\pm \sigma$ | I.3 | 3.0 | 3.0 | 4.0 | 4.0 |

Table 1 (continued)

| | I | 2 | 3 | 4 | 5 |
|---|---|---|---|---|---|
| Mean error of the arithmetic mean, $\pm m$ | 4.3 | I.3 | I.7 | I4 | I4 |
| Specific coagulation constant $t = \frac{M_1 - M_2}{\sqrt{m_1^2 + m_2^2}}$, mm | - | 5.0 | 4.5 | 5.3 | 5.5 |
| Certainty of the experiments, $\rho$ | - | $<$0.0I | $<$0.0I | $<$0.0I | $<$0.0I |

Table 1 (continued)

| Parameters | Coagulation index, $C_i = \frac{m}{R+K}$, mm | | | | |
|---|---|---|---|---|---|
| | I | 2 | 3 | 4 | 5 |
| Arithmetic mean, M | I.7 | 2.9 | 3.4 | 4.0 | 3.3 |
| Mean square deviation, $\pm \sigma$ | 0.6 | I.0 | 0.8 | I.4 | I.0 |
| Mean error of the arithmetic mean, $\pm m$ | 0.2 | 0.5 | 0.3 | 0.4 | 0.3 |
| Specific constant of coagulation $t = \frac{M_1 - M_2}{\sqrt{m_1^2 + m_2^2}}$, mm | - | 3.4 | 5.0 | 5.I | 4.6 |
| Certainty of the experiments, $\rho$ | - | $<$0.0I | $<$0.0I | $<$0.0I | $<$0.0I |

Note: 1 - Initial data.
2, 3, 4, 5 - 30, 60, 90 and 120 minuted after administration of the compound.

The data of Table 1 show that under the effect of the compund according to the present invention the parameters of the thromboelastogram are certainly change: R, K, R+K and C.

The compound according to the present invention in the dose of 25 mg/kg of the dag's bodymass provides a noticeable effect on thromboplastin activity and conversion of prothrombin into thrombin, substantially eccelerates the formation of a clot (the coagulation time K is reduced by 40-55%) and the total coagulation power of the blood (R+K), the factor C (coagulation index) is noticeable increased (by 71-133%).

The effect of the compound according to the present invention , viz. 3,1,18-trihydroxy-9,13-epoxylabdan-15-ic acid sodium salt trihydrate on hemostasis was studied in vitro with the use of a thromboelastogram. The results of the tests are shown in Table 2 hereinbelow.

As it is seen from the data of Table 2, the compound according to the present invention in the doses of 0.01, 0.1, 0.2 and 1.0 mg per 0.26 ml of blood substantially reduces the parameter R (the time of reaction of blood coagulation), I;E; accelerates the I and II phases of blood coagulation, the formation of active thromboplastin and conversion of prothrombin into thrombin.

In another series of experiments the effect of the compound according to the present invention was studied in biochemical tests.

Table 3 shows the data of the effect produced by the compound according to the present invention in the dose of 25 mg/kg of a dog's bodymass (intravenously).

Table 2

Effect of compound 3,16,18-trihydroxy-9,13-epoxylabdan-15-ic acid sodium salt trihydrate on thromboelastogram parameters (in vitro)

| Parameters | 3,16,18-trihydroxy-9,13-epoxylabdan-15-ic acid salt trihydrate, mg | | | | | |
|---|---|---|---|---|---|---|
| | 0.1 mg | | 0.2 mg | | 0.01 mg | |
| | K | O | K | O | K | O |
| Time of the reaction of blood coagulation, R, mm | II.0 | 8.0 | 25.0 | I2.0 | 26.0 | 7.0 |
| Coagulation index, C, mm | I9.6 | 20.8 | I7.0 | I7.4 | I7.0 | I8.0 |

| | 1 mg | | 5 mg | |
|---|---|---|---|---|
| | K | O | K | O |
| Time of the reaction of blood coagulation, R, mm | I3.0 | II.0 | I8.0 | 32.0 |
| Coagulation index, C, mm | 23.0 | 8.0 | I9.0 | 3.4 |

Note: K - control

O - experiment.

Table 3

Effect of 3,16,18-trihydroxy-9,13-epoxylabdan--15-ic acid sodium salt trihydrate on some parameters of the blood coagulation system (dose 25 mg/kg of the animal's bodymass, a group of 10 animals)

| Parameter | Time of blood coagulation, min | | | | |
|---|---|---|---|---|---|
| | I | 2 | 3 | 4 | 5 |
| Arithmetic mean, M | 5.I | 3.9 | 3.7 | 3.0 | 3.4 |
| Mean error of the arithmetic mean, $\pm \sigma$ | 0.20 | 0.I3 | 0.I6 | 0.I0 | 0.I5 |
| Specific constant of coagulation $t=\frac{M_1 - M_2}{\sqrt{m_1^2 + m_2^2}}$, mm | - | 9.2 | 8.7 | I9.0 | II.0 |
| Certainty of the experiments, | - | < 0.0I | < 0.0I | < 0.0I | < 0.0I |
| | Time of plasma recalcification, sec | | | | |
| | I | 2 | 3 | 4 | 5 |
| Arithmetic mean, M | II7.0 | 78.0 | 79.0 | 74.0 | 7I.0 |
| Mean error of the arithmetic mean, $\pm m$ | 5.0 | 2.8 | 3.3 | 2.5 | 3.0 |
| Specific constant of coagulation $t=\frac{M_1 - M_2}{\sqrt{m_1^2 + m_2^2}}$, mm | - | I4.0 | I2.0 | I7.0 | I5.0 |

Table 3 (continued)

| Parameter | I | 2 | 3 | 4 | 5 |
|---|---|---|---|---|---|
| Certainty of the experiments, $\rho$ | - | < 0.0I | < 0.0I | < 0.0I | < 0.0I |
| | Tolerance of the plasma to heparin, min | | | | |
| | I | 2 | 3 | 4 | 5 |
| Arithmetic mean, M | 6.8 | 4,8 | 4.6 | 4.3 | 4.I |
| Mean error of the arithmetic mean, $\pm m$ | 0.6 | 0.24 | 0.2 | 0.26 | 0.2I |
| Specific constant of coagulation $t=\frac{M_1 - M_2}{\sqrt{m_1^2 + m_2^2}}$, mm | - | 8.3 | II.0 | 9.6 | I3 |
| Certainty of the experiments, $\rho$ | - | < 0.0I | < 0.0I | < 0.0I | < 0.0I |
| | Trombin time, seconds | | | | |
| | I | 2 | 3 | 4 | 5 |
| Arithmetic mean, M | I7.0 | I2.0 | I2.0 | II.0 | II.0 |
| Mean error of the arithmetic mean, $\pm m$ | 0.6 | 0.4 | 0.4 | 0.4 | 0.4 |
| Specific constant of coagulation $t=\frac{M_1 - M_2}{\sqrt{m^2 + m^2}}$, mm | - | I2.0 | I2.0 | I4.0 | I2.0 |

Table 3 (continued)

| Parameter | I | 2 | 3 | 4 | 5 |
|---|---|---|---|---|---|
| Certainty of the experiments, $\rho$ | - | < 0.0I | < 0.0I | < 0.0I | < 0.0I |

Note: 1 - initial data.
2, 3, 4, 5 - 30, 60, 90 and 120 minutes after administration of the compound according to the present invention, viz. 3,16,18-trihydroxy-9,13-epoxylabdan-15-ic acid sodium salt trihydrate.

The data of Table 3 show that upon administration of the compound according to the present invention the blood coagulation time is shortened by 35-40%, the time of plasma recalcification - by 33-39%, the tolerance of plasma to heparin is increased by 30-40% and the thrombin time is reduced by 30-35%. The statistical processing of these tests has shown their certainty.

The study of the effect provided by the compound according to the present invention on the local blood hemorrhage has shown that the local effect of the compound in the concentration of 0.5% lowers the flood loss by 83% and the hemorrhage duration is reduced by 60%.

Therefore, the compound according to the present invention exhibits a local blood-erresting effect which makes it useful in parenchymatous bleedings, in operations on the brains, liver, spleen, kidney, bone tissue and the like. Furthermore, the compound according to the present invention increases the number of thrombocytes and activates adhesion and aggregation of thrombocytes.

The effect of the compound according to the present invention on heparin was also studied and it was shown that it did not prevent the effect of heparin but weakened it; tnis enables a conclusion that the compound of this invention can be used in the case of an overdosage of heparin and vice versa.

The compound according to the present invention, i.e. 3,16,18-trihydroxy-3,13-epoxylabdan-15-ic acid sodium salt trihydrate was tested for an acute toxicity. The experiments were carried out using white non-descipt mice with a bodymass of 18-20 g at an intraperitoneal administration of the compound according to the present invention in the doses of 100, 200, 400, 600, 800, 1,000 and 1,500 mg/kg of the animal's bodymass.

In the doses of 100, 200 mg/kg of the animal's bodymass in the behaviour of the test animals a short-time excitation was noted. In doses of 400-800 mg/kg of the animal's bodymass no sybstantial changes were obsarved in the behaviour of the test animals.

In the doses of 1,000 and 2,000 mg/kg of the animal's bodymass a light oppression was noted in the animal's behaviour, no death of the animals was observed.

The $LD_{50}$ of the compound according to the present invention was over 2,000 mg/kg of the animal's bodymass in the case of an intraperitoneal administration; consequently, the compound according to the present invention is non-toxic.

The compound according to the present invention was also studied for its chronical toxicity by administration thereof to animals (rats - a group of 40 animals, and to dogs - a group of 8 animals) in the doses of 10, 25 and 100 mg/kg of the animal's bodymass intravenously for the period of 10 days. The studies

have shown that upon a chronical administration the compound according to the present invention, viz. 3,16,18-trihydroxy-9,13-epoxylabdan-15-ic acid sodium salt trihydrate caused an insignificant increase in the number of leukocytes, whereas on the 10-th day the number of leukocytes returned to the initial values. The number of erythrocytes reduced after 5 administrations, no certain changes were observed on the part of hemoglobin and leukocytic formula.

In acute experiments in the case of an intravenous administration of the compound according to the present invention in the doses of 1, 5, 10, 25 and 50 mg/kg of the animal's bodymass, it did not cause negative changes in the central nervous system, cardiovascular system and respiration organs.

Pathomorphological investigations of the inner organs of the animals after a repeated administration of the claimed compound, in the doses of 10 and 25 mg/kg of the animal's bodymass during 10 days revealed no essential morphological and pathological changes. The compound according to the present invention neither provided any local irritation effect.

The compound according to the invention , viz. 3,18-dihydroxy-9,13-epoxylabdan-15-16-olide was administered to dogs (a group of 5 animals) in the dose of 50 mg/kg of the animal's bodymass hypodermally as a 20% oily solution. The results of the tests are shown in Table 4 hereinbelow.

As it follows from the Table 4, 3,18-dihydroxy-9,13-epoxylabdan-15,16-olide causes acceleration of blood coagulation. The blood coagulation time decreases by 51%, the time of recalcification - by 32%, the tolerance of the plasma to heparin was increased by 37%.

The $LD_{50}$ was determined on mice (a group of 30 animals with a bodymass of 20-21 g). The mice were administered with 3,18-dihydroxy-9,13-epoxylabdan-15,16-olide subcutaneously (a 20% oily solution). In the case of this manner of administration (i.e. hypodermal one) the $LD_{50}$ was equal to more than 2,000 mg/kg of the animal's bodymass.

Table 4

Effect of hypodermal administration of a 20% oily solution of 3,18-dihydroxy-9,13-epoxyl-abdan-15,16-olide on the process of blood coagulation in dogs

| Parameters | Dose of 50 mg/kg of the animal's bodymass | | | |
|---|---|---|---|---|
| | Prior to administration | Time after administration, min | | |
| | | 60 | 120 | 180 |
| 1 | 2 | 3 | 4 | 5 |
| Time of blood coagulation, seconds: | | | | |
| Arithmetic mean, M | 318.0 | 269.0 | 218.0 | 159.0 |
| Mean square deviation, ± | 28.0 | 24.0 | 34.0 | 33.0 |
| Mean error of the arithmetic mean, ± | - | 0.10 | 0.05 | 0.010 |
| Time of plasma recalcification, seconds: | | | | |
| Arithmetic mean, M | 106.0 | 102.0 | 84.0 | 72.0 |
| Mean square deviation, ± | 15.0 | 15.0 | 18.0 | 7.5 |
| Mean error of the arithmetic mean, ± | - | 0.8 | 0.7 | 0.1 |

Table 4 (continued)

| I | 2 | 3 | 4 | 5 |
|---|---|---|---|---|
| Tolerance of plasma to heparin, seconds: | | | | |
| Arithmetic mean, M | 2I4.0 | I92.0 | I38.0 | I35.0 |
| Mean square deviation, ± | 36.0 | 3I.0 | 4I.0 | 39.0 |
| Mean error of the arithmetic mean, ± | - | 0.6 | 0.I | 0.I |
| Fibrinogen, mg% | | | | |
| Arithmetic mean, M | 435.0 | 387.0 | 36I.0 | 293.0 |
| Mean square deviation, ± | I40.0 | I20.0 | I22.0 | 97.0 |
| Mean error of the arithmetic mean, ± | - | 0.3 | 0.6 | 0.3 |
| Fibrinolytic activity of blood, % | | | | |
| Arithmetic mean, M | 4I.0 | 53.0 | 54.0 | 43.0 |
| Mean square deviation, ± | 9.0 | I0.0 | I3.0 | 9.4 |
| Mean error of the arithmetic mean, ± | - | 0.I | 0.5 | 0.9 |

The pharmaceutical preparation according to the present invention was tested in clinic on human patients. The clinical studies were carried out on 292 surgical patients with various kinds of hemorrhages including gastro-intestinal hemorrages developing during the immediate post-operation days (94 patients), in erosive gastritis (16 patients), with mechanical jaundice: biliary calculi, cancer of the pancreas head, cancer of the portal fissure (6 patients), in the case of renal hemorrhage of a traumatic etiology (1 patient), after operations with an artificial blood circulation (at an excessive blood supply along the drains) on the occasion of the acquired and congenital vascular diseases (162 patients), as well as after operations of the coronary shunting.

Locally the preparation according to the present invention was administered in the case of bleeding from nasopharynx at a nasotracheal intubation (14 patients), in patients with bleeding wound surfaces on the body (5 patients). The patients' age varied from 16 to 60 years.

The preparation according to the present invention was introduced intravebously in the form of a 0.4, 0.5 and a 0.6% solution in bidistilled water in a single dose of from 0.05 to 0.1 g and maximum of up to 50-60 ml (0.25-0.3 g). The preparation according to the present invention was locally used in the tampons form of watted in it, as well as by way of treatment of the bleeding regions of the body with a solution of the preparation.

Control of the effectiveness of the preparation according to the present invention was made by clinical signs (discontinuation or decrease of the blood supply along the drains and stomach probe, as well as from nasopharinx), laboratory indications (content of hemoglobin, hemotacrite and erythrocytes) and by the values of the time of coagulation of blood and recalcification of plasma, the thrombin, prothrombin and heparin times, the content of fibrinogen and the degree of fibrinolytic activity.

The preparation according to the present invention was administered directly on the operation room, in the section of reanimation and in surgical department.

In the patients with nasal bleedings after a nasotracheal intobation the hemorrhages stopped after a single or two-time use of tampons wetted with the preparation according to the present invention.

In other patients with hemorrhages of the abovementioned etiology 30-40 minutes after administration of the preparation according to the present invention changes were noted on the part of the parameters of the coagulogram towards shortening of the time of the blood coagulation and the time of recalcification of plasma, reduction of the thrombin and heparin times, i.e. lowering of the degree of hypocoagulation which was accompanied by diminution of hemorrhagic signs.

In individual patients operated under conditions of an artificial blood circulation, after neutralization of heparin with protaminsulphate, low values of thrombocytes were observed which were possible to normalize by means of the preparation according to the present invention without recurring to transfusion of the thrombomass.

The preparation according to the present invention revealed no side effects upon its administration.

The pharmaceutical preparation according to the present invention and dicynone were subjected to a comparative study for their hemostatic effect. Twenty post-operation patients (cardiorevivification department). The preparations were administered in the

amount of 75 g. For the determination of the hemostatic effect determined by the functional state of the blood coagulation system the following characteristics were studied: thromboelastogram, the time of recalcification of citrate plasma and the plasma tolerance towards heparin. 20 minutes after the introduction of dicynone the parameter of the time of reaction of the rate of formation of thromboplastin was decreased by 32%, after administration of the preparation according to the present invention - by 75%, the time of coagulation was reduced, respectively, by 21 and 43%, the time of recalcification of plasma decreased by 43 and 61% respectively, tolerance of plasma to heparin increased by 36 and 52%, respectively (at an error of $p < 0.05$).

The study of hemostatic properties of the preparation according to the present invention was carried out in an express-diagnosis laboratory during operations on an "open heart" and in the department of cardiorevivification during the early post-operational period.

Immediately after switching-off the artificial blood-circulation apparatus the preparation according to the present invention was intravenously administered in the dose of 10 mg/kg to a group of 70 patients. 15 minutes thereafter the parameters of the blood coagulation system were studied.

The post-operational blood loss in the patients whom the preparation had not been administered was greater. Thus, an increased hemorrhage rate caused by disorders in the blood coagulation factors was noted in 5 patients out of the group of patients whom the preparation according to the present invention was administered and in 16 patients out of the group of patients whom the preparation according to the present

invention had not been administered (control group). Each group consisted of 70 patients; as regards the character of an operative intervention, duration of the artificial blood circulation and the degree of hypothermia the groups were similar.

The results of the tests of the hemostatic effect of the preparation according to the present invention are shown in Table 5 hereinbelow.

Contraindications against the administration of the preparation according to the present invention were thrombosis and liability to thromboembolic complications.

For a better understanding of the present invention, some specific examples illustrating preparation of the claimed compounds are given hereinbelow.

Example 1

Into a 4 l reactor there were charged 10 g of 3,15,16,18-tetrahydroxy-9,13-epoxylabdane, 2 l of toluene, added with 30 g of activated Raney nickel and a catalytical dehydrogenation was carried out at the temperature of 110°C. On completion of the process the catalyst was filtered-off and washed with toluene. The filtrate was twice treated with a 5% solution of caustic soda (by portions of 300 ml).

The alkaline solutions were combined and acidified with a 20% solution of sulfuric acid to an acidic reaction. The precipitate formed upon sedimentation was thrice extracted with diethyl ether (by portions of 1 l). The ethereal layer was washed to a neutral reaction. 3,18-dihydroxy-9,13-epoxylabdan-15,16-olide was obtained by recrystallization from diethyl ether of the residue after distilling-off the solvent - diethyl ether; the product m.p. was 143-144°C, chromatographic parameter $R_f$ = 0.76 (system: chloroform/methanol 10:2); specific rotation $[\alpha]_D$ = +13.82°

(C = 1.5, ethanol), yield of 20% as calculated for 3,15,16,18-tetrahydroxy-9,13-epoxylabdane. Molecular formula: $C_{20}H_{32}O_5$, molecular mass - 352. Calculated, %: C 68.18, H 9.09, O 22.73.

Found, %: C 68.12 (±0.10), H 9.11 (±0.10), O 22.71 (±0.10).

Table 5

Dynamics of variation of the parameters of the blood coagulation system defining the hemostatic effect of the preparation according to the present invention

| Parameters | Stages of study | | | |
|---|---|---|---|---|
| | 15 minutes after neutralization of heparin | | 1-2 hours after the operation | |
| | preparation of the invention administered in the dose of 10 mg/kg of the bodymass intravenously | Control group without administration of the preparation of the invention | Preparation of the invention administered in the dose of 10 mg/kg of the bodymass intravenously | Without administration of the preparation of the invention |
| 1 | 2 | 3 | 4 | 5 |
| Activated time of blood coagulation, seconds: | | | | |
| Arithmetic mean, M | I07.0 | II5.6 | I00.0 | I00.5 |
| Mean square deviation, ± σ | I2.3 | 30.2 | 5.2 | 6.I |
| Mean error of the arithmetic mean, ±m | 0.5 | 3.6 | 0.6 | 0.7 |

Table 5 (continued)

| I | 2 | 3 | 4 | 5 |
|---|---|---|---|---|
| Blood coagulation time, min. | | | | |
| Arithmetic mean, M | 6.3 | 8.4* | 6.0 | 8.0* |
| Mean square deviation, ± σ | 2.3 | 3.1 | 1.6 | 3.0 |
| Mean error of the arithmetic mean, ± m | 0.3 | 0.4 | 0.2 | 0.4 |
| Thrombin time, seconds | | | | |
| Arithmetic mean, M | 15.2 | 19.6* | 15.1 | 17.1* |
| Mean square deviation, ± σ | 9.8 | 5.1 | 2.0 | 4.0 |
| Mean error of the arithmetic mean, ±m | 0.5 | 0.6 | 0.2 | 0.5 |
| Prothrombin Index, % | | | | |
| Arithmetic mean, M | 81.0 | 72.4* | 88.3 | 81.3* |
| Mean square deviation, ± σ | 11.3 | 9.2 | 5.2 | 6.2 |
| Mean error of the arithmetic mean, ±m | 1.3 | 1.1 | 0.6 | 0.7 |
| Fibrinogen, mg% | | | | |
| Arithmetic mean, M | 226.3 | 192.3 | 286.3* | 245.1* |
| Mean square deviation, ± σ | 37.6 | 54.1 | 30.7 | 43.1 |
| Mean error of the arithmetic mean, ±m | 4.5 | 6.4 | 3.7 | 5.1 |

Note: * The difference between groups is certain (error $p < 0.05$).

PMR-spectrum

Singlets at 1.0 ppm and 0.93 ppm corresponded to methyl groups at quaternary atoms of C-4 carbon and C-10, while a doublet at 0.74 ppm pointed to the presence of a methyl group at C-17.

Within the range of 1.2-2.2 ppm 12 aliphatic methylene protons were located. Within the range of 2.35-3.0 ppm a quartet was located belonging to two protons. The spin-spin interaction constant (J = 17.0 Hz) showed that these methylene protons were located in the cycle adjacent to the C=O group. At 2.04 ppm a two-proton singlet was located; the chemical shift value depend on the substance concentration, thus pointing to its pertinence to protons of hydroxy groups. Within the range of 3.15-3.70 ppm the signals from protons were detected at C-18 and C-3. The quartet within the range of 4.0-4.4 ppm related to methylene protons at C-16 of the lactone cycle.

In the IR-spectrum there ware absorption bands at 3,250-3,400 $cm^{-1}$ (characteristic for hydroxy groups), 1,780 $cm^{-1}$ (five-membered lactone), 2,890-2,930 $cm^{-1}$ (methylene and methine groups).

Example 2

8,18-dihydroxy-9,13-epoxylabdan-15,16-olide was obtained in a manner similar to that described in the foregoing Example 1. Then 2 g of the product were dissolved in 10 ml of ethanol, alkanified with a 24% aqueous solution of caustic soda to the pH = 9. The solution was distilled-off, the residue was recrystallized from ethanol to give 3,16,18-trihydroxy-9,13-epoxylabdan-15-ic acid sodium salt trihydrate with the chromatographic parameter $R_f$ = 0.3 (system: chloroform/methanol 10:2), with the specific rotation $[\alpha]_D$= = -11.94 (C = 1 ethanol), the yield of 15% relative to 3,15,16,18-tetrahydroxy-9,13-epoxylabdane. The mole-

cular formula: $C_{20}H_{33}NaO_6$ $3H_2O$, molecular mass 446.5.

| | |
|---|---|
| Calculated, %: | C 53.75 |
| | H 8.8 |
| | O 32.23 |

| | | | |
|---|---|---|---|
| Found, %: | C 53.82 | H 8.93 | O 32.31 |
| | 53.64 | H 8.71 | 32.15 |

In the PMR-spectrum: a singlet was observed at 0.59 ppm, corresponding to a methyl group at $C_{10}$, a doublet at 0.8 ppm - to a methyl group at $C_8$ and a singlet at 0.84 ppm - to a methyl group at $C_4$. The quartet at 2.34-2.82 ppm corresponded to two methylene protons at $C_{14}$. The signals at 3.2-2.7 ppm corresponded to methylene protons at $C_{18}$ and $C_{16}$ and to a proton at $C_3$.

In the IR-spectrum there were absorption bands at 3,300-3,500 $cm^{-1}$ (characteristic for hydroxy groups), 1,550-1,610 $cm^{-1}$ and 1,400 $cm^{-1}$ (carboxy group), 2,850-2,950 $cm^{-1}$ (methylene and methine groups).

Example 3

3,18-duhydroxy-9,13-epoxylabdan-15,16-olide was obtained in a manner similar to that described in the foregoing Example 1, except that benzene was used as organic solvents during catalytical dehydrogenation.

The yield of the end product was 7%. Melting point 142-143°C. Chromatographic parameter $R_f$ = 0.76 (system: chloroform/methanol 10:2). Specific rotation $[\alpha]_D$ = +13.82 (C = 1.5, ethanol). Molecular formula $C_{20}H_{33}O_5$.

| | |
|---|---|
| Calculated, %: | C 68.18 |
| | H 9.09 |
| | O 22.75 |
| Found, %: | C 68.14 |
| | H 9.10 |
| | O 22.73 |

PMR-spectrum:

$CH_3$ at $C_4$ = 1.0 ppm (singlet)
$CH_3$ at $C_{10}$ = 0.93 ppm (singlet)
$CH_3$ at $C_{17}$ = 0.74 ppm (douplet)
12 - $CH_2$ = 1.2-2.2 ppm (multiplet)

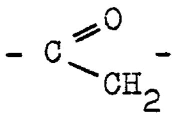 = 2.35-3.0 ppm (quartet)

$-CH_2-O-$ = 4.0-4.4 ppm (quartet)

IR-spectrum had absorption bands at 3250-3400 $cm^{-1}$ (characteristic for hydroxy groups), 1780 $cm^{-1}$ (five-membered lactone), 2890-2930 $cm^{-1}$ (methylene and methine groups).

Example 4

3,18-dihydroxy-9,13-epoxylabdan-15,16-olide was obtained in a manner similar to that described in Example 1, except that acetone was used as organic solvent during catalytical dehydrogenation. Melting point 143-144°C. Chromatographic parameter $R_f$ = 0.76 (system: chloroform/methanol 10:2). Yield of the end product was 5%. Specific rotation $[\alpha]_D$ = +13.82°. (C = 1.5, ethanol). Molecular formula $C_{20}H_{32}O_5$.

| | | |
|---|---|---|
| Calculated, %: | C | 68.18 |
| | H | 9.09 |
| | O | 22.72 |
| Found, %: | C | 68.13 |
| | H | 9.11 |
| | O | 22.74 |

PMR-spectrum:

$CH_3$ at $C_4$ = 1.0 ppm (singlet)
$CH_3$ at $C_{10}$ = 0.93 ppm (singlet)
$CH_3$ at $C_{17}$ = 0.74 ppm (doublet)
12-$CH_3$ = 1.2-2.2 ppm (multiplet)

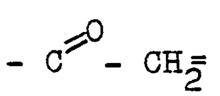 2.35-3.0 ppm (quartet)

$-CH_2O-$ = 4.0-4.4 ppm (quartet)

The IR-spectrum has absorption bands at 3,250-3,400 $cm^{-1}$ (characteristic for hydroxy groups), 1,780 $cm^{-1}$ (five-membered lactone), 2,890-2,930 $cm^{-1}$ (methylene and methine groups).

Example 5

Into a 2 l reactor there were charged 10 g of 3,15,16,18-tetrahydroxy-9,13-epoxylabdane, 250 ml of acetone, 150 ml of distilled water and 1.5 ml of a concentrated sulfuric acid. The mass was stirred, extracted for 6 times with cyclohexane by portions of 500 ml. The cyclohexane extracts were washed to a neutral reaction and cyclohexane was distilled-off, while from the residue 3,18-dihydroxy-9,13-epoxylabdan-15,16-olide was obtained in a manner similar to that described in Example 1 hereinbefore, except that the desired product in the form of 3,18-dihydroxy-9,13-epoxylabdan-15,16-olide was isolated in the following manner. The residue after distilling-off the solvent (9 g) and filtering-off the catalyst was dissolved in 50 ml of ethanol, added with 15 ml of a 7% solution of sulphuric acid and the mixture was heated at reflux for 30 minutes, whereafter operations for isolation of the desired product were carried out as described in Example 1, except that after the addition of caustic soda the by-products were extracted with chloroform (twice by portions of 100 ml). The yield of the end product was 50%. The melting point was 142-143°C. The chromatographic parameter $R_f$ = 0.76 (system: chloroform/methanol 10:2). The specific rotation $[\alpha]_D$ = +13.82° (C = 1, 5, ethanol). The molecular formula $C_{20}H_{32}O_5$. The molecular mass - 352.

Calculated, %: C 68.18
H 9.09
O 22.73

Found, %: C 68.13
H 9.12
O 22.70.

PMR-spectrum:

$CH_3$ at $C_4$ = 1.0 ppm (singlet)
$CH_3$ at $C_{10}$ = 0.93 ppm (singlet)
$CH_3$ at $C_{17}$ = 0.74 ppm (doublet)
12-$CH_2$ = 1.2-2.2 ppm (multiplet)

$-C^{=O}$ - $CH_2$ = 2.35-3.0 ppm (quartet)

$-CH_2-O-$ = 4.0-4.4 ppm (quartet).

The IR-spectrum had absorption bands at 3,250-3,400 $cm^{-1}$ (characteristic for hydroxy groups), 1,780 $cm^{-1}$ (five-membered lactone), 2,890-2,930 $cm^{-1}$ (methylene and methine groups).

## Industrial Applicability

The compounds according to the present invention exhibit a hemostatic activity and can be useful in medicine as a pharmaceutical preparation employed for various forms of hemorrhages.

# C L A I M S :

1. Derivatives of 3,18-dihydroxy-9,13-epoxylabdane of the general formula:

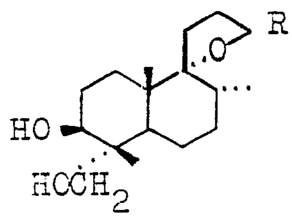

wherein:

R = 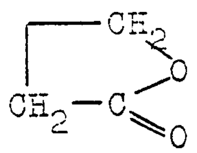 ; 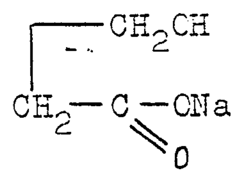 $\cdot 3H_2O$

2. A method for preparing derivatives of 3,18-dihydroxy-9,13-epoxylabdane according to Claim 1, characterized in that 3,15,16,18-tetrahydroxy-9,13-epoxylabdane is subjected to hydrogenation in the presence of a catalyst in a medium of a boiling organic solvent, followed by isolation of the desired product.

3. A method according to Claim 2, characterized in that 3,15,16,18-tetrahydroxy-9,13-epoxylabdane prior to said catalytical dehydrogenation is treated with acetone in the presence of a 2% aqueous solution of sulphuric acid.

4. A method according to Claims 2 and 3, characterized in that as the dehudrogenation catalyst an activated Raney nickel is used.

5. A method according to Claims 2 to 4, characterized in that as the organic solvent toluene is used.

6. A hemostatic-effect pharmaceutical preparation comprising an active principle and a pharmaceutically acceptable solvent, characterized in that as the active principle it incorporates 3,16,18-trihydroxy-9,13-epoxylabdan-15-ic acid sodium salt trihydrate of the following formula:

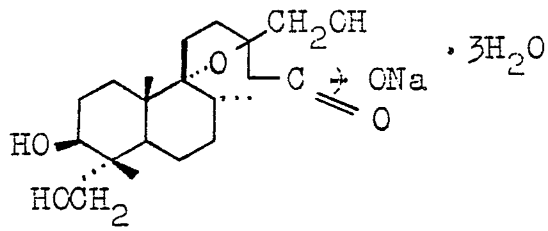

according to Claim 1.

7. A pharmaceutical preparation according to Claim 6 in the form of an injection solution, characterized in that it contains the active principle in an amount of from 0.4 to 0.6% by mass.

8. A pharmaceutical preparation according to Claims. 6 and 7, characterized in that as the pharmaceutical solvent it contains bidistilled water.

# INTERNATIONAL SEARCH REPORT

International Application No PCT/SU 88/00252

**I. CLASSIFICATION OF SUBJECT MATTER** (if several classification symbols apply, indicate all) [3]

According to International Patent Classification (IPC) or to both National Classification and IPC

IPC[5] C 07 D 443/IO, 307/94, A 6I K 9/08, 35/78, 3I/I9, 3I/34

**II. FIELDS SEARCHED**

Minimum Documentation Searched [4]

| Classification System | Classification Symbols |
|---|---|
| IPC[4] | C 07 D 493/IO, 307/94, A 6I K 9/08, 35/78, 3I/34 |

Documentation Searched other than Minimum Documentation to the Extent that such Documents are Included in the Fields Searched [5]

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| A | Izvestia Akademii nauk SSSR, chemical series,No. 9, I970, Nauka (Moscow), O.S. Chizhov et al, "Struktura lagokhilina", pp. I983-I99I (noted in the description) | I |
| A | Khimia prirodnykh soedineny,N I, I976. FAN UzSSR, (Tashkent), Z.I.Mavlankulova et al, "3,I8,O-Izopropilidenlagokhilin iz Lagochilus pubescens", pp. II3-II4 | I-5 |
| A | US, A, 4503240 (CONSORTIUM FÜR ELEKTROCHEMISCHE INDUSTRIELLE GmbH), March 5, I985 (05.03.85), cf. claims<br>& DE, AI, 3240054,03.05.84<br>EP, BI, I07857, 30.I2.86<br>JP, A, 5908238I, I2.05.84 | I-8 |
| A | SU, AI, 27I7I9, (I.E.AKOPOV et al), March 2, I976 )(02.03.76) | I-8 |
| A | SU, AI, 99459 (M.M.ABRAMOV), December I954, (12.54), cf. Claims | I-8 |
| A | EP, A2, 0I65458 (T.HASEGAWA COMPANY, LTD.), December 27, I985 (27.I2.85), cf. Claims<br>& JP, A, 6023948I, 28.II.85 | I-8 |

* Special categories of cited documents: [15]

"A" document defining the general state of the art

"E" earlier document but published on or after the international filing date

"L" document cited for special reason other than those referred to in the other categories

"O" document referring to an oral disclosure, use, exhibition or other means

"P" document published prior to the international filing date but on or after the priority date claimed

"T" later document published on or after the international filing date or priority date and not in conflict with the application, but cited to understand the principle or theory underlying the invention

"X" document of particular relevance

**IV. CERTIFICATION**

| Date of the Actual Completion of the International Search [2] | Date of Mailing of this International Search Report [2] |
|---|---|
| August 3, I989 (03.08.89) | August I5, I989 (I5.08.89) |
| **International Searching Authority [1]** | **Signature of Authorized Officer [20]** |
| ISA/SU | N.Shepelev |